# EUROPEAN PATENT SPECIFICATION

(11) **EP 4 579 680 B1**
(45) Date of publication and mention of the grant of the patent: **23.09.2026**
(21) Application number: 24223279.1
(22) Date of filing: 25.12.2024
(51) Int. Cl.: G16H 40/63, G01N 35/02, G01N 35/00

(54) **SAMPLE ANALYSIS SYSTEM AND SAMPLE INFORMATION DISPLAY METHOD**
PROBENANALYSESYSTEM UND PROBENINFORMATIONSANZEIGEVERFAHREN
SYSTÈME D'ANALYSE D'ÉCHANTILLON ET PROCÉDÉ D'AFFICHAGE D'INFORMATIONS D'ÉCHANTILLON

(30) Priority: 25.12.2023 CN 202311798514; 11.11.2024 CN 202411605029
(43) Date of publication of application: 02.07.2025
(73) Proprietor: Shenzhen Mindray Bio-Medical Electronics Co., Ltd., Shenzhen, Guangdong 518057 (CN)
(72) Inventor: YE, Bo, Shenzhen, 518057 (CN); ZHENG, Wenbo, Shenzhen, 518057 (CN); RAO, Linshang, Shenzhen, 518057 (CN)
(74) Representative: KIPA AB

(56) References cited:
- WO-A1-2017/132168
- US-A1- 2023 097 384

## Description

### TECHNICAL FIELD

The disclosure relates to the field of medical detection technologies, and in particular, to a sample analysis system, a sample information display method, an electronic device, and a storage medium.

### BACKGROUND

Abnormal quality of a blood sample may affect accuracy of test results of the blood sample. For example, a chylous sample may cause a pseudo high hemoglobin measurement result, and a blood clot may cause an abnormality in sample aspiration. Blood sample quality detection is an important part of clinical laboratory blood sample detection. In order to control quality of a blood sample, after obtaining test results by a sample testing pipeline, when finding an abnormal test result, it is common practice for a clinician or laboratory technician to leave a sample result reviewing region to manually find the blood sample, and then visually determine whether the quality of the blood sample is abnormal. In this process, it is time-consuming and labor-consuming to manually find a blood sample, and efficiency of reviewing blood samples is low.

WO 2017/132168 A1 discloses an automated specimen testing apparatus with a track and a quality-check module that acquires multi-view images of specimen containers, processes them into a 3D model and uses this model for pre-analytical quality assessment (e.g. volume, artefacts, HIL indices).

US 2023/097384 A1 discloses a biological specimen analysis device that rotates a labelled sample tube, generates a developed (unwrapped) view of the tube surface, and determines specimen type and solution volume from this image while compensating for label influence.

### SUMMARY

The invention is defined by the appended claims.

In view of this, embodiments of the disclosure provide a sample analysis system, a sample information display method, an electronic device, and a storage medium.

In order to achieve the above objective, technical solutions of the disclosure are implemented as follows.

An embodiment of the disclosure provides a sample analysis system, including:
an input module, a transfer track, an analysis device, an image obtaining module, a processor, and a display screen; wherein
the input module is configured to carry a sample container loaded with a blood sample, and transfer the sample container to the transfer track;
the transfer track is configured to transfer the sample container to the analysis device;
the analysis device is configured to analyze the blood sample in the sample container, to obtain an analysis result;
the image obtaining module is configured to capture at least two first images of the sample container, which are different from each other, before the analysis device performs a sample analysis on the blood sample, where each first image at least partially displays at least part of the blood sample in the sample container;
the processor is configured to generate, based on the at least two first images, a rotatable three-dimensional representative of the sample container containing the blood sample, and/or an animation for displaying from different angles the sample container containing the blood sample;
the display screen is configured to display the analysis result and output the rotatable three-dimensional representative and/or output the animation on a user interface.

An embodiment of the disclosure further provides a sample analysis system, including an input module, a transfer track, an analysis device, an image obtaining module, a processor, and a display screen; wherein
the input module is configured to carry a sample container loaded with a blood sample, and transfer the sample container to the transfer track;
the transfer track is configured to transfer the sample container to the analysis device;
the analysis device is configured to analyze the blood sample in the sample container, to obtain an analysis result;
the image obtaining module is configured to capture at least two first images of the sample container, which are different from each other, before the analysis device performs a sample analysis on the blood sample;
the processor is configured to perform image stitching on the at least two first images, to obtain a third image;
the display screen is configured to display the analysis result and output the third image on a user interface.

An embodiment of the disclosure further provides a sample information display method, including:
obtaining at least two first images of a sample container loaded with a blood sample, where each first image at least partially displays at least part of the blood sample in the sample container, and the at least two first images are obtained by imaging the sample container before an analysis device performs a sample analysis on the blood sample;
generating, based on the at least two first images, a rotatable three-dimensional representative of the sample container containing the blood sample, and/or an animation for displaying from different angles the sample container containing the blood sample; and
controlling a display screen to display an analysis result of the blood sample in the sample container, and to output the rotatable three-dimensional representative and/or the animation, on a user interface.

An embodiment of the disclosure further provides an electronic device, including a processor, and a memory configured to store a computer program runnable on the processor. The processor is configured to run the computer program to perform the steps of any one of above methods.

An embodiment of the disclosure further provides a storage medium with a computer program stored therein. When the computer program is executed by a processor, the steps of any one of above methods are implemented.

In the sample analysis system, the sample information display method, the electronic device, and the storage medium provided in the embodiments of the disclosure, the sample analysis system includes the input module, the transfer track, the analysis device, the image obtaining module, the processor, and the display screen. The input module is configured to carry the sample container loaded with the blood sample, and transfer the sample container to the transfer track. The transfer track is configured to transfer the sample container to the analysis device. The analysis device is configured to analyze the blood sample in the sample container, to obtain the analysis result. The image obtaining module is configured to capture the at least two first images of the sample container, which are different from each other, before the analysis device performs the sample analysis. The processor may be configured to generate, based on the at least two first images, the rotatable three-dimensional representative of the sample container containing the blood sample, and/or an animation for displaying from different angles the sample container containing the blood sample, or may be configured to perform image stitching on the at least two first images, to obtain the third image. The display screen is configured to display the analysis result and output the rotatable three-dimensional representative and/or the animation and/or the third image on the user interface. According to the above solutions, a user may review whether the blood sample is abnormal based on the analysis result of the blood sample outputted by the display screen on the user interface, so that efficiency of reviewing the blood sample is improved. The user may further check or re-check whether the analysis result is accurate based on the three-dimensional representative and/or the animation and/or the third image outputted by the display screen on the user interface for displaying the sample container from a plurality of angles, in particular, from lateral angles, so that accuracy and reliability of the review result of the blood sample can be improved. Sample information for the sample container may be further obtained by using the third image, so that accuracy and success rate in identification of the sample information can be improved.

### BRIEF DESCRIPTION OF THE DRAWINGS

FIG. 1 is a schematic diagram of an architecture of a sample analysis system according to an embodiment of the disclosure;
FIG. 2 is an example diagram of rotary imaging according to an embodiment of the disclosure;
FIG. 3 is an example diagram of rotary imaging according to another embodiment of the disclosure;
FIG. 4 is an example diagram of performing frame interpolation on first images according to an embodiment of the disclosure;
FIG. 5 is an example diagram of performing image processing on a first image according to an embodiment of the disclosure;
FIG. 6 is an example diagram of performing image processing on a second image according to an embodiment of the disclosure;
FIG. 7 is an example diagram of performing image processing on a first image according to another embodiment of the disclosure;
FIG. 8 is an example diagram of performing image processing on a second image according to another embodiment of the disclosure;
FIG. 9 is a schematic flowchart of a sample information display method according to an embodiment of the disclosure; and
FIG. 10 is a schematic diagram of a structure of an electronic device according to the embodiment of the disclosure.

### DESCRIPTION OF THE PREFERRED EMBODIMENTS

In order to make objectives, technical solutions, and advantages of the disclosure clearer, the disclosure will be further described below in detail in conjunction with the accompanying drawing and the embodiments. It should be understood that the specific embodiments described herein are merely intended to explain the disclosure but are not intended to define the disclosure.

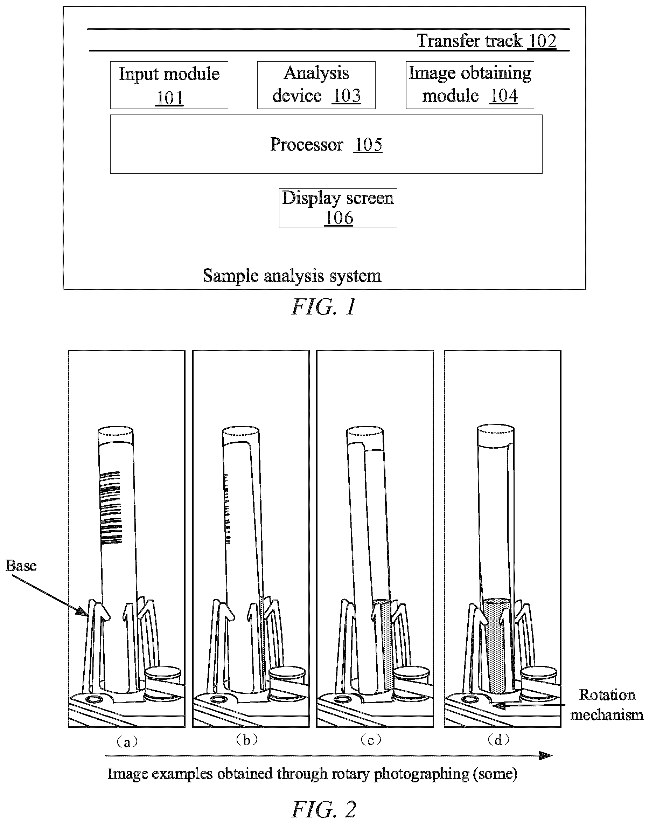

FIG. 1 is a schematic diagram of an architecture of a sample information display system according to an embodiment of the disclosure. As shown in FIG. 1, the sample analysis system includes an input module 101, a transfer track 102, an analysis device 103, an image obtaining module 104, a processor 105, and a display screen 106. The processor 105 may separately communicate with the image obtaining apparatus 104, the analysis device 103, and the display screen 106.

The input module 101 is configured to carry a sample container loaded with a blood sample, and transfer the sample container to the transfer track 102.

The transfer track 102 is configured to transfer the sample container to the analysis device 103.

The analysis device 103 is configured to analyze the blood sample in the sample container, to obtain an analysis result.

The image obtaining module 104 is configured to capture at least two first images of the sample container, which are different from each other, before the analysis device 103 performs the sample analysis. For example, each first image at least partially displays at least part of the blood sample in the sample container.

The processor 105 is configured to generate, based on the at least two first images, a rotatable three-dimensional representative of the sample container containing the blood sample, and/or an animation for displaying from different angles the sample container containing the blood sample.

The display screen 106 is configured to display the analysis result and output the rotatable three-dimensional representative and/or output the animation on the user interface.

Herein, the input module 101 transfers the carried sample container to the transfer track 102, the image obtaining apparatus 104 captures the at least two first images of the sample container, the transfer track 102 transfers the sample container to the analysis device 103, and the analysis device 103 may analyze the blood sample in the sample container, to obtain the analysis result. In an embodiment, the analysis device 103 includes a sample component, a reagent component, and a measurement component. Specifically, after the transfer track 102 transfers the sample container to the analysis device 103, the sample component of the analysis device 103 aspirates the blood sample from the sample container, and provides the aspirated blood sample to the measurement component of the analysis device 103. The reagent component of the analysis device 103 aspirates a reagent, and provides the aspirated reagent to the measurement component of the analysis device 103. The measurement component of the analysis device 103 uniformly mixes the blood sample provided by the sample component and the reagent provided by the reagent component to prepare a reaction solution, incubates the reaction solution, and analyzes the reaction solution that is incubated, to obtain the analysis result of the blood sample in the sample container. The analysis device 103 may send the analysis result to the processor 105 and/or the display screen 106 when obtaining the analysis result of the blood sample in the sample container, or may send the analysis result to the processor 105 and/or the display screen 106 when the analysis result of the blood sample in the sample container does not satisfy a preset condition. The set condition indicates a condition that a normal blood sample satisfies. If the analysis result satisfies the preset condition, it indicates that the blood sample is a normal sample; or if the analysis result does not satisfy the preset condition, it indicates that the blood sample is abnormal, for example, the blood sample is at least one of a hemolytic sample, a jaundice sample, and a lipemic sample.

For each sample container, the image obtaining apparatus 104 may capture at least two first images of the sample container that are different from each other before the analysis device performs sample analysis, where each first image at least partially displays at least part of a blood sample in the sample container. For example, the image obtaining apparatus 104 may capture the at least two first images of the sample container during rotation before the analysis device performs the sample analysis on the blood sample. Capturing the at least two first images of the sample container during rotation may be understood as capturing the at least two first images of the sample container during rotation of the sample container about a vertical central axis or about a vertical direction, that is, performing rotary scanning or rotary imaging on the same sample container. The at least two first images of the sample container captured by the image obtaining apparatus 104 include first images captured from different viewing angles or different imaging angles. The image obtaining apparatus 104 may directly transfer all first images of the sample container to the processor 105. Alternatively, the image obtaining apparatus may transfer all first images of the sample container to another device for storage, and the processor 105 may obtain the first images of the sample container from the another device. Further, the image obtaining apparatus 104 stores the captured first images of the sample container to a preset storage path of a camera 103 or another electronic device, and the processor 105 obtains the first images of the sample container from the preset storage path, and stores the first images. In actual application, a corresponding folder may be created for each sample container, and the first images of the sample container are stored in the corresponding folder, so that first images of different sample containers may be distinguished. In addition, the image obtaining apparatus 104 stores all captured first images of one sample container to the preset storage path at a time, the processor 105 may clear all the first images under the preset storage path after the processor 105 obtains all the first images of the sample container from the preset storage path, and then the image obtaining apparatus 104 stores all captured first images of another sample container to the set storage path. In this way, sample containers are read one by one for images.

In actual application, a sample container may be understood as a transparent or translucent container for loading a blood sample, and may be a blood collection tube, a testing tube, a sample tube, a sampling tube, a test tube, a bottle, a sample cup, etc. The sample container is loaded with the blood sample, which may be a serum sample or a plasma sample, and reagents for detection may be added to the blood sample. A surface of the sample container is affixed or attached with a label for marking or recording related information of the blood sample. The label may include identification information of the blood sample in the sample container, such as a barcode, or a sequence code combining letters and/or numbers.

The processor 105 obtains the at least two first images of the same sample container captured by the image obtaining apparatus 104, and generates, based on the at least two first images obtained, the rotatable three-dimensional representative of the sample container containing the blood sample, and/or an animation for displaying from different angles the sample container containing the blood sample. Herein, the rotatable three-dimensional representative may be used to display the sample container containing the blood sample from a plurality of angles, especially lateral angles. Herein, the animation consists of a plurality of image frames.

In an embodiment, the processor 106 may be further configured to generate, as the rotatable three-dimensional representative, a three-dimensional image or a three-dimensional model of the sample container containing the blood sample, based on the at least two first images obtained. For example, the three-dimensional image may be obtained by performing image stitching on the at least two first images.

In an example, when obtaining many first images corresponding to a same sample container, the processor 105 may determine the obtained first images as image frames for forming the animation of the sample container; or when obtaining few first images corresponding to a same sample container, the processor 105 may perform frame interpolation on the first images, to obtain image frames for forming the animation of the sample container. In actual application, the processor 105 may preprocess the obtained first images of the same sample container, and generate, based on preprocessed first images, image frames for forming the animation of the sample container. A preprocessing operation may include cropping the first images to reduce size of the first images, so that storage resources are saved. The preprocessing operation may further include denoising, to improve image sharpness. After generating the image frames that correspond to the sample container and that are used to form the animation, the processor 105 may store the image frames that correspond to the sample container and that are used to form the animation, and may further generate, based on the image frames that correspond to the sample container and that are used to form the animation, the animation for displaying the sample container in rotation, for example, stitch, in a preset sequence, the image frames for forming the animation, to obtain the animation for displaying the sample container in rotation. The image frames stored by the processor 105 are used for the display screen 106 to generate, based on the image frames that correspond to the sample container and that are used to form the animation, the animation for displaying the sample container during rotation.

In an embodiment, the processor 105 may further instruct or control the display screen 106 to display the three-dimensional representative and/or the animation on the user interface, for example, control the display screen 106 to display the three-dimensional representative in rotation on the user interface, in response to a first set instruction. The display screen 106 displays the analysis result of the blood sample in the sample container, and outputs the three-dimensional representative and/or the animation corresponding to the same sample container, on the user interface. The analysis result displayed by the display screen 106 may be sent by the processor 105 or the analysis device 103. There is a correspondence or an association relationship between the three-dimensional representative and/or the animation of the sample container and the analysis result of the blood sample in the sample container. For example, the three-dimensional representative and/or the animation of the sample container and the analysis result corresponding to the same sample container may be associated by using information indicated by the label affixed to the sample container. For example, the display screen 106 may obtain the animation for displaying the sample container during rotation, which is generated in advance by the processor 105, and output, on the user interface, the animation generated in advance by the processor 105. For another example, the display screen 106 may obtain the image frames that are stored by the processor 105, that correspond to the sample container and that are used to form the animation, generate, based on the obtained image frames for forming the animation, the animation for displaying the sample container during rotation, and output, on the user interface, the animation generated in real time. The three-dimensional representative and/or the animation outputted by the display screen 106 is used for a related person to determine or check whether sample quality of the blood sample in the sample container is abnormal.

It should be noted that the processor 105 and the display screen 106 may be arranged in a same device, or may be separately arranged in different devices. For example, the processor 105 and the display screen 106 are arranged in a same terminal device. For another example, the processor 105 is arranged in a server or a control device, and the display screen 106 is arranged in a terminal, or the display screen 106 is used as an independent display device.

As described above, the image obtaining apparatus 104 may capture the at least two first images of the sample container during rotation before the analysis device performs the sample analysis. In order to enable a user to observe the sample container from different angles or viewing angles, rotary imaging may be performed on the sample container, to obtain images of the sample container from different angles or viewing angles. Based on this, in an embodiment, the image obtaining apparatus 104 includes a camera, a rotation mechanism, and a gripping mechanism.

The gripping mechanism is configured to grip an end portion of the sample container, to keep the sample container in a vertical state.

The rotation mechanism is configured to drive the gripping mechanism to rotate, to drive the sample container gripped by the gripping mechanism to rotate about a vertical direction.

The camera is configured to capture the at least two first images of the sample container during rotation.

Herein, the rotation mechanism is connected to the gripping mechanism, and the gripping mechanism may grip a bottom or a top of the sample container. The rotation mechanism may drive the sample container gripped by the gripping mechanism to rotate about a vertical direction at a preset speed, for example, rotate 360 degrees. The camera captures the at least two different first images of the sample container during rotation at a preset frame rate. The camera is an industrial camera. In actual application, a duration of imaging the sample container by the camera may be greater than or equal to a duration required by the sample container to perform one revolution or rotate 360 degrees, so that the camera can obtain first images of the sample container corresponding to different angles or viewing angles during one revolution of the sample container.

Considering that the camera is prone to motion artifacts when imaging a moving object, in a rotary imaging scenario, in order to ensure that the camera of the image obtaining apparatus 104 can obtain clear images of the sample container from different angles during one revolution of the sample container, further, there is a preset correspondence or mapping relationship between an exposure duration and/or a imaging frequency of the camera and a rotational speed of the rotation mechanism.

Different rotational speeds of the rotation mechanism correspond to different exposure durations, and/or different rotational speeds of the rotation mechanism correspond to different imaging frequencies. The rotational speed of the rotation mechanism may be determined based on a speed of identifying the label on the sample container. Certainly, the rotational speed of the rotation mechanism may be a fixed preset rotational speed. The exposure duration may also be understood as exposure time. In order to ensure that the camera can capture a clear image, the exposure duration of the camera is less than or equal to a quotient of accuracy of the camera and the rotational speed of the rotation mechanism. The accuracy of the camera may also be referred to as pixel accuracy, which is equal to a quotient of a unidirectional field of view and a unidirectional resolution of the camera. In actual application, the imaging frequency may be replaced with a frame rate. The frame rate may be understood as the number of image frames captured per second. For example, the frame rate is 20 frames per second.

In an embodiment, the gripping mechanism includes:
a base configured to grip the bottom of the sample container; and/or
a manipulator configured to grip the top of the sample container.

Herein, the image obtaining apparatus 104 may grip the bottom of the sample container through the base, to keep the sample container in the vertical state, drive the base through the rotation mechanism to rotate, to drive the sample container to rotate about the vertical direction, and perform rotary imaging on the sample container. The base may be a base for accommodating a single tube. In other words, the base of the image obtaining apparatus 104 is configured to grip one sample. The base may also be referred to as a sample base. FIG. 2 shows an example in which the image obtaining apparatus 104 drives the base through the rotation mechanism to rotate for rotary imaging.

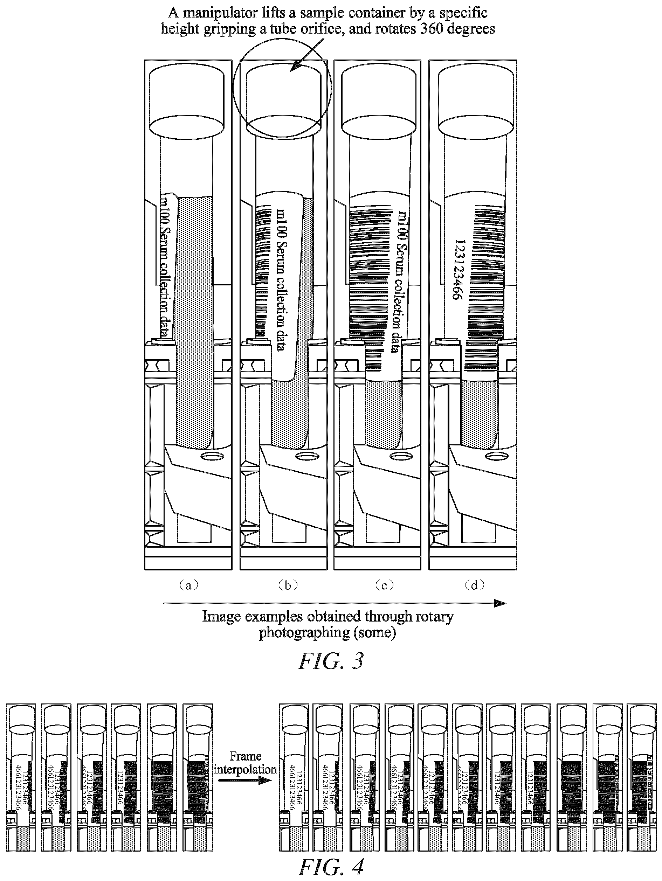

The image obtaining apparatus 104 may further grip the top of the sample container via the manipulator, lift the sample container by a specific height, drive the manipulator through the rotation mechanism to rotate, to drive the sample container gripped by the manipulator to rotate about the vertical direction, and perform rotary imaging on the sample container. FIG. 3 shows an example in which the image obtaining apparatus 104 drives the manipulator through the rotation mechanism to rotate for rotary imaging.

It should be noted that in an application scenario of pipeline detection for blood samples, for example, pipeline detection for biochemical immunoassay or blood cell pipeline detection, the image obtaining apparatus 104 may be arranged on the transfer track 102 for conveying sample containers.

In another embodiment, the image obtaining apparatus 104 may capture at least two first images of a fixedly placed sample container from different lateral angles before the analysis device performs sample analysis. For example, the image obtaining apparatus 104 may rotate circumferentially about the fixedly placed sample container, to image, from different lateral angles, the sample container loaded with a blood sample. For another example, the image obtaining apparatus 104 may include a plurality of cameras arranged circumferentially around the fixedly placed sample container, to image, from different lateral angles, the sample container loaded with a blood sample.

In an embodiment, the processor 105 may be further configured to find a target image from the at least two first images, where a preset parameter value of the target image is the largest among the at least two first images, and is greater than a preset threshold. Accordingly, the display screen is further configured to display the target image on the user interface.

Therefore, the target image, which displays the blood sample best, among the at least two first images can be obtained and outputted, to help the user determine the quality of the blood sample.

Further, the processor 105 is further configured to control the display screen 106 to display the three-dimensional representative, e.g. the three-dimensional image in rotation on the user interface if the target image cannot be found from the at least two first images.

In other words, if the target image that satisfies a condition cannot be found, the three-dimensional representative, e.g. the three-dimensional image is automatically rotated, so that the user can observe the sample container displayed at various angles, to determine the quality of the blood sample.

In an embodiment, the preset parameter value represents a ratio of a first area representing an area of a first image, that is occupied by the blood sample, to a second area representing an area of said first image, that is occupied by the sample container. Herein, the target image, in which a proportion of the blood sample is the largest and is greater than the preset threshold, is selected from the at least two first images.

As some implementations, the processor 105 may be configured to input the at least two first images into a pre-trained neural network model, such as a convolutional neural network model, to determine whether one of the input first images is the target image.

As some other implementations, the processor 105 may be configured to find the target image from the at least two first images based on an image feature-based image processing algorithm. For example, the processor 105 automatically extracts one or more of an edge feature, a grayscale distribution feature, a color feature, a texture feature, a shape feature, and a spatial relationship feature of each image, and then identifies the target image based on the extracted image feature.

In order to enable the user to observe the sample container from various angles, in an embodiment, the processor 105 generating, based on the at least two first images, an animation for displaying from different angles the sample container containing the blood sample includes:
performing frame interpolation on the at least two first images, to obtain image frames for forming the animation.

Herein, the processor 105 may perform frame interpolation on the obtained first images by using a preset frame interpolation algorithm, to obtain the image frames for forming the animation of the sample container, thereby generating, based on the image frames of the same sample container, the animation for displaying the sample container in rotation. As shown in FIG. 4, the processor 105 may perform frame interpolation on every two first images, to obtain a transition frame between the corresponding two first images, also referred to as an intermediate frame, and determine the first images and the transition frame as the image frames for forming the animation of the sample container.

The processor 105 may further preprocess the first images, for example, crop and denoise the first images, before performing frame interpolation on the first images.

Considering that some sample containers are circular cylinders with surface curvatures, a surface curvature of the sample container needs to be removed before frame interpolation. Based on this, in an embodiment, the processor 105 performing frame interpolation on the at least two first images includes:
performing first image processing on each first image, to obtain at least two second images; performing frame interpolation on the at least two second images, to obtain at least three second images; and performing second image processing on each of the at least three second images, to obtain the image frames for forming the animation, where
the first image processing is used at least to remove the surface curvature of the sample container in each first image, and the second image processing is used at least to recover the surface curvature of the sample container in each second image.

Herein, the processor 105 performs first image processing on each first image to remove the surface curvature of the sample container in said first image, to obtain a second image corresponding to said first image, where one first image corresponds to one second image; performs frame interpolation on the obtained second images by using the preset frame interpolation algorithm, to obtain a plurality of second images; and performs second image processing on each second image to recover the surface curvature of the sample container in said second image, to obtain the image frames for forming the animation of the sample container.

In actual application, the processor 105 may obtain the image frames for forming the animation of the sample container in the following manner.

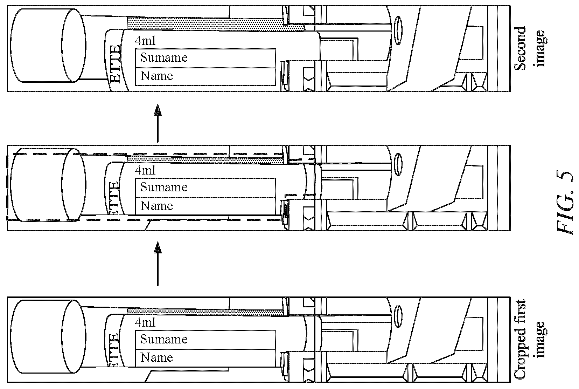

A target region, in which the sample container is located, in each first image is determined, and said first image is cropped based on the boundary of the target region, to obtain a first image including the target region. The sample container in the cropped first image is determined, and first image processing is performed on the cropped first image by using a set image processing algorithm, to remove the surface curvature of the sample container in the cropped first image, thereby obtaining a corresponding second image. FIG. 5 shows an example of the cropped first image and the second image, and there is one first image and one second image in FIG. 5.

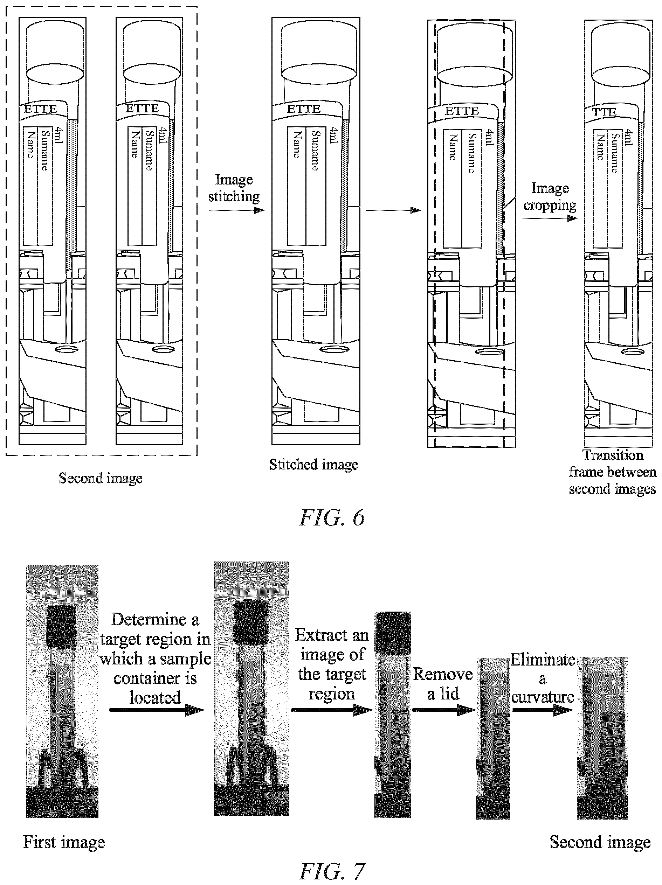

Second images corresponding to every two adjacent first images are selected as an image pair, and the following operations are performed on each image pair: extracting at least one type of image feature of each second image in the image pair by using the preset image processing algorithm; determining same or similar pixels in the image pair based on the at least one type of image feature of each second image in the image pair; determining a stitching position of the two second images in the image pair based on the same or similar pixels in the image pair; performing image stitching on the two second images in the image pair based on the determined image stitching position, to obtain a stitched image; and cropping the stitched image to obtain at least one transition frame between the two second images in the image pair, where a size of the transition frame obtained through cropping is the same as that of the second images in the image pair, and the transition frame obtained through cropping is also referred to as a second image. FIG. 6 shows an example of generating a corresponding transition frame by using two second images. The at least one type of image feature includes at least one of the following: an edge feature, a grayscale distribution feature, a color feature, a texture feature, a shape feature, and a spatial relationship feature.

Second image processing is performed on the second images in the image pair and the corresponding transition frame, to recover the surface curvature of the sample container in the corresponding images, thereby obtaining the image frames for forming the animation of the sample container.

In an embodiment, the processor 105 may be configured to receive, from the user interface, the first set instruction entered by the user, that is, the first set instruction is entered by the user through the user interface.

As some implementations, in order to display the three-dimensional representative, e.g. the three-dimensional image or play the animation on the user interface, the user may click on a "play" option or button on the user interface.

As some other implementations, the processor 105 may alternatively control the display screen 106 to display the three-dimensional representative, e.g. the three-dimensional image interactively on the user interface. In this way, the user may directly operate, for example, click on or drag, the three-dimensional representative displayed on the user interface, to rotate the three-dimensional representative on the user interface, so that the user can observe, from different angles, the sample container represented by the three-dimensional representative.

For example, when the display screen 106 displays the interactive three-dimensional representative, e.g. the three-dimensional image on the user interface, the user may click on the three-dimensional representative with a mouse, to enter the first set instruction, and the processor 105 controls, in response to the first set instruction, the display screen 106 to automatically rotate the three-dimensional representative on the user interface, to display the sample container from various angles. In addition, the user may further click on a stop button or click on the three-dimensional representative again with the mouse, and the processor 105 controls, in response to the further instruction, the display screen 106 to stop rotating the three-dimensional representative on the user interface and to display a current image at which rotation is stopped.

In another embodiment, the first set instruction may be generated when the analysis result obtained by the analysis device 103 does not satisfy a preset condition.

As some implementations, the processor 105 is further configured to control the display screen 106 to output prompt information on the user interface when the analysis result does not satisfy the preset condition, where the prompt information is used to prompt the user to enter the first set instruction through the user interface.

Herein, when determining that the analysis result of the blood sample in the sample container does not satisfy the preset condition, or receiving the analysis result that is sent by the analysis device 103 and that does not satisfy the preset condition, the processor 105 controls the display screen 106 to output the prompt information on the user interface, so that the user enters the first set instruction through the user interface, to view the three-dimensional representative, e.g. the three-dimensional image, or the animation of the sample container corresponding to the analysis result and further determine whether the blood sample in the sample container is abnormal. There is a correspondence or an association relationship between the analysis result and the identification information of the blood sample recorded by the label outside the sample container. The prompt information may include the identification information (such as barcode) of the blood sample corresponding to the analysis result.

In this embodiment, in an application scenario of reviewing sample quality, when the analysis result of the blood sample in the sample container is abnormal, the related person only needs to review the sample quality of the blood sample based on the three-dimensional representative, e.g. the three-dimensional image, or the animation corresponding to the sample container, to determine whether the blood sample is abnormal. Therefore, only when the analysis result of the blood sample in the sample container is abnormal, the display screen 106 outputs the prompt information on the user interface, to prompt the user to enter the first set instruction through the user interface, and then the processor 105 instructs, in response to the first set instruction, the display screen 106 to output the three-dimensional representative, e.g. the three-dimensional image, or the animation of the sample container on the user interface. This can reduce data processing of the display screen and reduce workload of the related person in reviewing sample quality, so that sample quality reviewing efficiency is improved.

As some other implementations, the processor 105 may be further configured to generate the first set instruction when the analysis result does not satisfy the preset condition.

In order to improve sample quality reviewing efficiency, in an embodiment, the processor 105 is further configured to, when the analysis result does not satisfy the preset condition, control the display screen 106 to output, on the user interface, prompt information indicating that the analysis result does not satisfy the preset condition, the three-dimensional representative, and optionally the target image, and/or the animation.

In order to help the related person observe the blood sample in the sample container, in an embodiment, the display screen 106 outputting the animation on the user interface includes:
playing the animation one or more times on the user interface; and/or
displaying a preset image frame of the animation on the user interface after playing of the animation is paused or completed.

Herein, the display screen 106 may play the animation one or more times on the user interface in an automatic play mode or a manual play mode, and/or, display the preset image frame of the animation on the user interface after playing of the animation is paused or completed. Playing the animation for a plurality of times may be understood as playing the animation continuously for a plurality of times or repeatedly. The blood sample in the sample container may be observed in the preset image frame.

For example, the display screen 106 automatically plays the animation on a preset user interface when the preset user interface is open or displayed. For another example, the display screen 106 plays the animation or pauses playing of the animation on the user interface when detecting a play instruction, where the playback instruction may be entered through a touchscreen of the display screen 106, or an external device of the display screen 106, such as a keyboard, the mouse, and a microphone.

In order to help the related person observe the blood sample in the sample container, in an embodiment, a first parameter value of the preset image frame is greater than a first set threshold, where
the first parameter value represents a ratio of a first area representing an area of the preset image frame, that is occupied by the blood sample, to a second area representing an area of the preset image frame that is occupied by the sample container.

Herein, the first area may also be understood as an area of a window or region, which is not covered by the label on the surface of the sample container, in the preset image frame. The preset image frame may be an image frame whose first parameter value is the largest among the image frames for forming the animation. In other words, the window, which is not covered by the label on the surface of the sample container, in the preset image frame is the largest.

In an embodiment, at least one image parameter of the preset image frame meets a preset parameter requirement, where the at least one image parameter includes at least one of the following:
brightness;
sharpness; and
contrast.

Herein, the preset image frame meeting a preset image quality parameter requirement is selected based on the at least one image parameter, so that image quality of the preset image frame and sharpness of an image of the blood sample included in the preset image frame can be ensured. This helps the user manually review the sample quality of the blood sample or re-check the analysis result, thereby improving accuracy and reliability of the review result of the blood sample.

In an embodiment, the processor 105 is further configured to:
control the display screen 106 to play the animation on the user interface, in response to a second set instruction entered by the user through the user interface; and/or
control the display screen 106 to stop playing the animation on the user interface, in response to a third set instruction entered by the user through the user interface; and/or
control the display screen 106 to display image frames of the animation frame by frame or in a frame skipping manner on the user interface, in response to a fourth set instruction entered by the user through the user interface.

Herein, different set instructions correspond to different play modes or output modes for the animation, the display screen 106 supports in playing the animation in different modes, and a set instruction may be understood as a human-machine interaction instruction. The user may trigger different set instructions in different operation manners, so that the processor 105 controls the display screen 106 to play the animation, stop playing the animation, or stop at an image frame, thereby helping the user find the best image frame to review sample quality. The best image frame includes a clear image of the blood sample.

Based on the above embodiments, the display screen in the sample analysis system may display the analysis result of the blood sample in the sample container and the three-dimensional representative and/or the animation of the sample container, and the user may review, based on the analysis result of the blood sample outputted by the display screen on the user interface, whether the blood sample is abnormal, without visually determining whether quality of the blood sample is abnormal, so that efficiency in reviewing the blood sample may be improved. In addition, whether the analysis result is accurate may be further checked or re-checked by using the three-dimensional representative and/or the animation of the sample container when the analysis result indicates that the blood sample is abnormal, so that accuracy and reliability of the review result of the blood sample can be improved.

With continued reference to FIG. 1, an embodiment of the disclosure further provides another sample analysis system having the following differences from the sample analysis system provided in the above embodiments.

The processor 105 is configured to perform image stitching on the at least two first images, to obtain a third image.

The display screen 106 is configured to display the analysis result and output the third image on the user interface.

Herein, the image obtaining module 104 captures the at least two first images of the sample container, which are different from each other, before the analysis device 103 performs sample analysis, where for example each first image at least partially displays at least part of the blood sample in the sample container. Each first image may further include a partial image of a barcode and/or a sequence code outside the sample container. The processor 105 obtains the at least two first images of the same sample container captured by the image obtaining apparatus 104, and performs image stitching on the at least two first images of the same sample container, to obtain a third image. The display screen 106 displays the analysis result of the blood sample in the same sample container, and outputs the third image corresponding to the sample container, on the user interface. The third image may be understood as a large image or a tiled image. The third image may include an image of the blood sample in the sample container and/or a complete image of the barcode and the sequence code outside the sample container. The third image is obtained by performing image stitching on the first images based on an image feature of a target region, in which the sample container is located, in each first image of the same sample container. For example, the image of the blood sample in the sample container included in the third image is obtained by performing image stitching on images of target regions based on an image feature of the target region, in which the sample container is located, in each first image of the same sample container. The complete image of the barcode and the sequence code outside the sample container included in the third image is obtained by performing image stitching on partial images of the barcode and/or the sequence code outside the sample container based on an image feature of the target region, in which the sample container is located, in each first image of the same sample container.

Specifically, the processor 105 may determine the target region, in which the sample container is located, in each of the at least two first images of the same sample container; crop said first image based on the boundary of the target region, to obtain a first image including the target region, and extract at least one type of image feature of each cropped first image; determine same or similar pixels in every two adjacent first images based on the at least one type of image feature of the every two adjacent first images; determine a stitching position of the every two adjacent first images based on the same or similar pixels in the every two adjacent first images; and perform image stitching based on the stitching position of the every two adjacent first images, to obtain a third image.

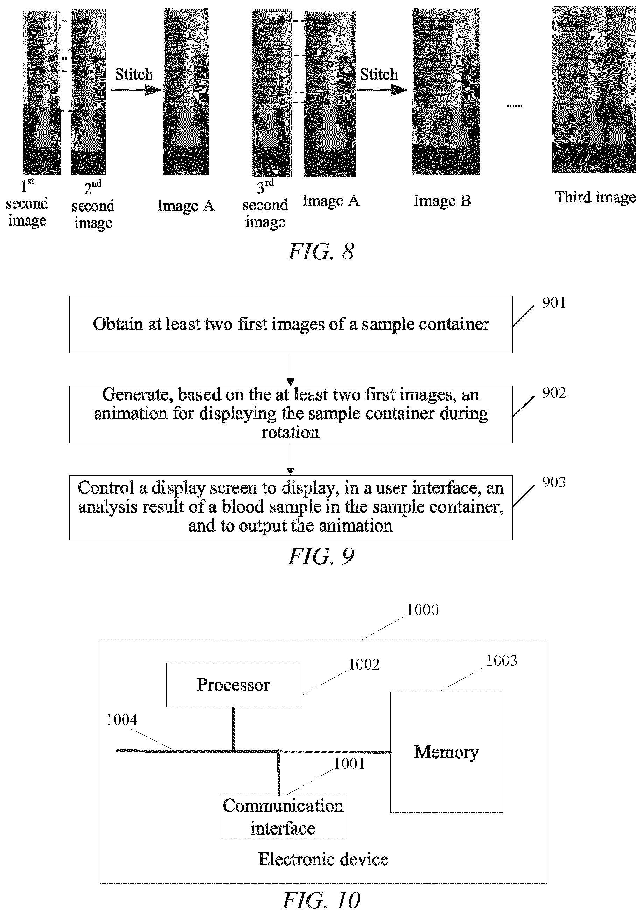

In actual application, considering that some sample containers are circular cylinders with surface curvatures, a surface curvature of the sample container needs to be removed before stitching. As shown in FIG. 7 and FIG. 8, the processor 105 performs image stitching on the at least two first images of the same sample container, to obtain a third image, as follows.

As shown in FIG. 7, the processor 105 determines the target region, in which the sample container is located, in each first image of the same sample container, and crops the first image based on the boundary of the target region, to obtain the first image including the target region; and determines the sample container in the cropped first image, and performs first image processing on the cropped first image by using a preset image processing algorithm, to remove the surface curvature of the sample container in the cropped first image, thereby obtaining a corresponding second image. It should be noted that the processor 105 may further perform frame interpolation on at least two second images, to obtain at least three second images.

As shown in FIG. 8, the processor 105 sorts the second images corresponding to the first images of the same sample container based on a sequence in which the image obtaining module 104 obtains the at least two first images of the same sample container; extracts at least one type of image feature of each of the first two second images by using a preset image processing algorithm, and determines same or similar pixels in the first two second images based on the at least one type of image feature of each second image; determines a stitching position of the first two second images based on the same or similar pixels in the first two second images; and performs image stitching on the first two second images based on the determined image stitching position, to obtain a stitched image A. Then, the processor extracts at least one type of image feature of the image A and at least one type of image feature of a 3^{rd} second image by using the preset image processing algorithm; determines same or similar pixels in the image A and the 3^{rd} second image based on the at least one type of image feature of the image A and the at least one type of image feature of the 3^{rd} second image; determines a stitching position of the image A and the 3^{rd} second image based on the same or similar pixels in the image A and the 3^{rd} second image; performs image stitching on the image A and the 3^{rd} second image based on the determined image stitching position, to obtain a stitched image B; determines a stitching position of the image B and a 4^{th} second image based on same or similar pixels in the image B and the 4^{th} second image; and performs image stitching on the image B and the 4^{th} second image based on the determined image stitching position, to obtain a stitched image C. By analogy, a final third image is obtained when all the second images of the sample container are traversed.

It should be noted that when determining a stitching position of two images, the processor 105 may calculate a displacement between the two images based on the stitching position of the two images, and perform image stitching on the two images based on the stitching position of the two images and the displacement.

In an embodiment, the processor 105 performing image stitching on the at least two first images includes:
performing image stitching on the at least two first images, to obtain a rotatable three-dimensional image as the third image.

Herein, the rotatable three-dimensional image may be used to display the sample container containing the blood sample from a plurality of angles, especially lateral angles.

Herein, for implementation of the rotatable three-dimensional image, reference is made to the above related descriptions, and details are not described herein again.

Based on the above embodiment, the display screen in the sample analysis system may display the analysis result of the blood sample in the sample container and the third image of the same sample container, and a user may review, based on the analysis result of the blood sample outputted by the display screen on the user interface, whether the blood sample is abnormal, without visually determining whether quality of the blood sample is abnormal, so that efficiency in reviewing the blood sample can be improved. When the third image includes the image of the sample in the sample container, whether the analysis result is accurate may be further checked or re-checked by using the third image if the analysis result indicates that the blood sample is abnormal, so that accuracy and reliability of the review result of the blood sample can be improved. When the third image includes the complete image of the barcode and the sequence code outside the sample container, it is also convenient for the user to view the barcode and the sequence code outside the sample container, and sample information may also be successfully identified by using the complete image of the barcode and the sequence code included in the third image, so that accuracy and success rate in obtaining the sample information for the sample container are improved.

Based on the related descriptions of the above embodiments, correspondingly, an embodiment of the disclosure further provides a sample information display method applied to an electronic device. The electronic device includes only the processor 105 in the above embodiments, or may include the processor 105 and the display screen 106 in the above embodiments. The electronic device may generate a three-dimensional representative and/or an animation for displaying a sample container. Referring to FIG. 9, the method includes the following steps.

Step 901: Obtaining at least two first images of a sample container loaded with a blood sample, where the at least two first images are obtained by imaging the sample container before an analysis device performs sample analysis. For example, the at least two first images are obtained by imaging the sample container during rotation before the analysis device performs sample analysis.

In an embodiment, each first image at least partially displays at least part of the blood sample in the sample container.

Herein, the electronic device obtains the at least two first images of the same sample container captured by the above image obtaining apparatus 104. The at least two first images may be obtained by the image obtaining apparatus 104 by imaging the same sample container, for example, by imaging the same sample container during rotation, before the analysis device 103 performs sample analysis. The at least two images of the same sample container captured by the image obtaining apparatus 104 include images captured from different viewing angles or different imaging angles.

Step 902: Generating, based on the at least two first images, a rotatable three-dimensional representative, e.g. a three-dimensional image of the sample container containing the blood sample, and/or an animation for displaying from different angles the sample container containing the blood sample, for example, generating an animation for displaying the sample container during rotation.

Herein, for implementation process of step 902, reference is made to the above implementation process in which the processor 105 generates the three-dimensional representative and/or the animation of the sample container. Details are not described herein again.

Step 903: Controlling a display screen to display an analysis result obtained by the analysis device by analyzing the blood sample in the sample container, and to output the rotatable three-dimensional representative and/or output the animation, on a user interface.

In order to improve accuracy and reliability of the review result of the blood sample and to improve accuracy and success rate in obtaining sample information for the sample container, additionally or alternatively, in an embodiment, the method further includes:
performing image stitching on the at least two first images, to obtain a third image; and
controlling the display screen to display the analysis result of the blood sample in the sample container, and to output the third image, on the user interface.

Herein, for implementation of obtaining the third image, reference is made to the above related descriptions, and details are not described herein again.

In an embodiment, the method further includes:
in response to a first set instruction, controlling the display screen to display the three-dimensional representative and/or the animation on the user interface, for example, to display the three-dimensional image in animation, and optionally, controlling the display screen to display the three-dimensional representative, e.g. the three-dimensional image in rotation on the user interface.

In an example, the first set instruction is entered by a user through the user interface. Herein, the user may enter the first set instruction through the user interface based on an actual need, and the electronic device controls, in response to the first set instruction, the display screen to output, on the user interface, the three-dimensional representative and/or the animation for displaying the sample container, for example, the sample container in rotation, so that the user can observe whether the blood sample in the sample container is abnormal. This may reduce data processing of the processor.

In an embodiment, the method further includes:
controlling the display screen to output prompt information on the user interface when the analysis result does not satisfy a preset condition, where the prompt information is used to prompt the user to enter the first set instruction through the user interface.

In an embodiment, the method further includes:
when the analysis result does not satisfy a preset condition, automatically generating the first set instruction, or controlling the display screen to output, on the user interface, prompt information indicating that the analysis result does not satisfy the preset condition, and the three-dimensional representative and/or the animation.

In an embodiment, the method further includes:
finding a target image from the at least two first images, where a preset parameter value of the target image is the largest among the at least two first images, and is greater than a preset threshold; and
controlling the display screen to display the target image on the user interface.

In an embodiment, the method further includes:
controlling the display screen to display the three-dimensional representative in rotation on the user interface if the target image cannot be found from the at least two first images.

In an embodiment, the preset parameter value represents a ratio of a first area representing an area of a first image, that is occupied by the blood sample, to a second area representing an area of said first image, that is occupied by the sample container.

In an embodiment, controlling the display screen to output the animation on the user interface includes:
playing the animation one or more times on the user interface; and/or
displaying a preset image frame of the animation on the user interface after playing of the animation is paused or completed.

In an embodiment, a first parameter value of the preset image frame is greater than a first set threshold, where
the first parameter value represents a ratio of a first area representing an area of the preset image frame, that is occupied by the blood sample, to a second area representing an area of the set image frame that, is occupied by the sample container.

In an embodiment, at least one image parameter of the preset image frame meets a preset parameter requirement, where the at least one image parameter includes at least one of the following:
brightness;
sharpness; and
contrast.

In an embodiment, after controlling the display screen to output the animation on the user interface, the method further includes:
controlling the display screen to play the animation on the user interface, in response to a second set instruction entered by the user through the user interface; and/or
controlling the display screen to stop playing the animation on the user interface, in response to a third set instruction entered by the user through the user interface; and/or
controlling the display screen to display image frames of the animation frame by frame or in a frame skipping manner on the user interface, in response to a fourth set instruction entered by the user through the user interface.

In an embodiment, generating, based on the at least two first images, an animation for displaying from different angles the sample container containing the blood sample includes:
performing frame interpolation on the at least two first images, to generate the animation for displaying the sample container during rotation.

Herein, for implementation process in which the electronic device generates the animation of the sample container, reference is made to the above related descriptions, and details are not described herein again.

In an embodiment, performing frame interpolation on the at least two first images includes:
performing first image processing on each first image, to obtain at least two second images, where the first image processing is used at least to remove a surface curvature of the sample container in each first image;
performing frame interpolation on the at least two second images, to obtain at least three second images; and
performing second image processing on each of the at least three second images, to obtain image frames for forming the animation, where the second image processing is used at least to recover the surface curvature of the sample container in each second image.

Herein, for implementation process in which the electronic device performs frame interpolation on the at least two first images, reference is made to the above related descriptions, and details are not described herein again.

In an embodiment, generating, based on the at least two first images, a rotatable three-dimensional representative of the sample container containing the blood sample includes:
generating, as the three-dimensional representative, a rotatable three-dimensional image or a rotatable three-dimensional model of the sample container containing the blood sample, based on the at least two first images.

Based on the above embodiment, the electronic device may display the analysis result of the blood sample in the sample container and the three-dimensional representative and/or the animation of the same sample container, on the user interface, and the user may review, based on the analysis result of the blood sample output in the user interface, whether the blood sample is abnormal, without visually determining whether quality of the blood sample is abnormal, so that efficiency in reviewing the blood sample can be improved. In addition, whether the analysis result is accurate may be further checked or re-checked by using the three-dimensional representative and/or the animation of the sample container when the analysis result indicates that the blood sample is abnormal, so that accuracy and reliability of the review result of the blood sample can be improved.

In order to implement the method corresponding to FIG. 9, an embodiment of the disclosure further provides an electronic device. As shown in FIG. 10, the electronic device 1000 includes:
a communication interface 1001 capable of exchanging information with another network node; and
a processor 1002 connected to the communication interface 1001 to exchange the information with the another network node and configured to run a computer program to perform the method provided in one or more technical solutions from the electronic device side, where the computer program is stored on a memory 1003.

Specifically, the processor 1002 is configured to: obtain at least two first images of a sample container; generate, based on the at least two first images, a rotatable three-dimensional representative of the sample container containing a blood sample, and/or an animation for displaying from different angles the sample container containing the blood sample, for example, generate an animation for displaying the sample container during rotation; and control a display screen to display an analysis result of the blood sample in the sample container, and to output the three-dimensional representative and/or the animation, on a user interface, where the at least two first images are obtained by imaging the sample container before an analysis device performs sample analysis.

In an alternative and additional embodiment, the processor 1002 is further configured to: perform image stitching on the at least two first images, to obtain a third image; and control the display screen to display the analysis result of the blood sample in the sample container, and to output the third image, on the user interface.

In an embodiment, the processor 1002 is further configured to control the display screen to output the three-dimensional representative and/or the animation on the user interface, in response to a first set instruction entered by a user through the user interface.

In an embodiment, the processor 1002 is further configured to control the display screen to output prompt information on the user interface when the analysis result does not satisfy a preset condition, where the prompt information is used to prompt the user to enter the first set instruction through the user interface.

In an embodiment, the processor 1002 is further configured to: when the analysis result does not satisfy the preset condition, control the display screen to output prompt information indicating that the analysis result does not satisfy the preset condition, and the three-dimensional representative and/or the animation, on the user interface.

In an embodiment, the processor 1002 is specifically configured to: play the animation one or more times in the user interface; and/or
display a preset image frame of the animation on the user interface after playing of the animation is paused or completed.

In an embodiment, a first parameter value of the preset image frame is greater than a first set threshold, where
the first parameter value represents a ratio of a first area representing an area of the preset image frame, that is occupied by the blood sample, to a second area representing an area of the preset image frame, that is occupied by the sample container.

In an embodiment, at least one image parameter of the preset image frame meets a preset parameter requirement, where the at least one image parameter includes at least one of the following:
brightness;
sharpness; and
contrast.

In an embodiment, the processor 1002 is further configured to: control the display screen to play the animation on the user interface, in response to a second set instruction entered by the user through the user interface; and/or
control the display screen to stop playing the animation on the user interface, in response to a third set instruction entered by the user through the user interface; and/or
control the display screen to display image frames of the animation frame by frame or in a frame skipping manner on the user interface, in response to a fourth set instruction entered by the user through the user interface.

In an embodiment, the processor 1002 is specifically configured to perform frame interpolation on the at least two first images, to generate the animation for displaying the sample container during rotation.

In an embodiment, the processor 1002 is specifically configured to: perform first image processing on each first image, to obtain at least two second images; perform frame interpolation on the at least two second images, to obtain at least three second images; and perform second image processing on each of the at least three second images, to obtain image frames for forming the animation, where the first image processing is used at least to remove a surface curvature of the sample container in each first image; the second image processing is used at least to recover the surface curvature of the sample container in each second image.

It should be noted that specific processing of the processor 1002 and the communication interface 1001 may be understood with reference to the above method.

Certainly, in actual application, various components of the electronic device 1000 are coupled together via a bus system 1004. It may be understood that the bus system 1004 is configured to implement connection and communication between these components. In addition to a data bus, the bus system 1004 further includes a power bus, a control bus, and a status signal bus. However, for clarity of description, various buses are marked as the bus system 1004 in FIG. 10.

The memory 1003 in embodiments of the disclosure is configured to store various types of data to support the operation of the electronic device 1000. Examples of such data include any computer program for the operation on the electronic device 1000.

The method disclosed in the foregoing embodiment of the disclosure may be applied to the processor 1002, or may be implemented by the processor 1002. The processor 1002 may be an integrated circuit chip having a signal processing capability. During implementation, each step of the method may be completed by an integrated logic circuit of hardware in the processor 1002 or an instruction in the form of software. The above processor 1002 may be a general-purpose processor, a digital signal processor (DSP) or other programmable logic devices, a discrete gate or a transistor logic device, a discrete hardware component, or the like. The processor 1002 may implement or perform various methods, steps, and logic block diagrams disclosed in the embodiments of the disclosure. The general-purpose processor may be a microprocessor, any conventional processor, or the like. The steps of the method disclosed in conjunction with the embodiments of the disclosure may be directly performed by a hardware decoding processor, or performed by a combination of hardware and software modules in a decoding processor. The software module may be located in a storage medium located in the memory 1003, and the processor 1002 reads the information in the memory 1003, and completes the steps of the above method in conjunction with its hardware.

In an exemplary embodiment, the electronic device 1000 may be implemented by one or more application-specific integrated circuits (ASICs), DSPs, programmable logic devices (PLDs), complex programmable logic devices (CPLDs), field programmable gate arrays (FPGAs), general-purpose processors, controllers, microcontroller units (MCUs), microprocessors (microprocessors), or other electronic components for performing the above method.

It may be understood that the memory (memory 1003) in embodiments of the disclosure may be a volatile memory or a non-volatile memory, or may include both a volatile memory and a non-volatile memory. The non-volatile memory may be a read-only memory (ROM), a programmable read-only memory (PROM), an erasable programmable read-only memory (EPROM), an electrically erasable programmable read-only memory (EEPROM), a ferromagnetic random access memory (FRAM), a flash Memory, a magnetic surface memory, an optical disc, or a compact disc read-only memory (CD-ROM). The magnetic surface memory may be a disk memory or a magnetic tape memory. The volatile memory may be a random access memory (RAM), which acts as an external cache. By way of example and not limitation, many forms of RAMs may be used, such as a static random access memory (SRAM), a synchronous static random access memory (SSRAM), a dynamic random access memory (DRAM), a synchronous dynamic random access memory (SDRAM), a double data rate synchronous dynamic random access memory (DDR SDRAM), an enhanced synchronous dynamic random access memory (ESDRAM), a synclink dynamic random access memory (SLDRAM), and a direct rambus random access memory (DR RAM). The memory described in the embodiments of the disclosure is intended to include, but not limited to, these memories and any other suitable types of memories.

In an exemplary embodiment, an embodiment of the disclosure further provides a storage medium, such as a computer storage medium, specifically a computer-readable storage medium, for example, including the memory 1003 storing a computer program that may be executed by the processor 1002 of the electronic device 1000 to perform the steps of the method from the electronic device side. The computer-readable storage medium may be a memory such as a FRAM, a ROM, a PROM, an EPROM, an EEPROM, a flash memory, a magnetic surface memory, an optical disc, or a CD-ROM.

It should be noted that "first", "second", and the like are used to distinguish between similar objects but do not necessarily indicate a specific order or sequence.

The term "and/or" herein describes only an association relationship between associated objects and represents that three relationships may exist. For example, A and/or B may represent that the following three cases exist: Only A exists, both A and B exist, and only B exists. In addition, the term "at least one" herein indicates any one or a combination of at least two of multiple. For example, including at least one of A, B, and C may represent including any one or more elements selected from a set including A, B, and C.

In addition, the technical solutions specified in the embodiments of the disclosure may be combined in any manner if no conflict is caused.

The above descriptions are merely preferred embodiments of the disclosure, and are not intended to limit the scope of protection as defined by the appended claims.

## Claims

1. A sample analysis system, comprising an input module, a transfer track, an analysis device, an image obtaining module, a processor, and a display screen, wherein
the input module is configured to carry a sample container loaded with a blood sample, and transfer the sample container to the transfer track;
the transfer track is configured to transfer the sample container to the analysis device;
the analysis device is configured to analyze the blood sample in the sample container, to obtain an analysis result;
the image obtaining module is configured to capture at least two first images of the sample container, which are different from each other, before the analysis device performs a sample analysis on the blood sample, wherein each first image at least partially displays at least part of the blood sample in the sample container;
the processor is configured to generate, based on the at least two first images, a rotatable three-dimensional representative of the sample container containing the blood sample and/or an animation for displaying from different lateral angles the sample container containing the blood sample; and
**characterized in that**:
the display screen is configured to display the analysis result and output the rotatable three-dimensional representative and/or the animation on a user interface;
wherein the processor is further configured to: in response to a first set instruction entered by a user through the user interface, control the display screen to display the three-dimensional representative in rotation on the user interface, so as to display the sample container containing the blood sample from a plurality of lateral angles, and/or the processor is further configured to: in response to a second set instruction entered by the user through the user interface, control the display screen to play the animation on the user interface, so as to display the sample container containing the blood sample from a plurality of lateral angles.

2. The sample analysis system of claim 1, **characterized in that** the processor is further configured to:
control the display screen to output prompt information on the user interface when the analysis result does not satisfy a preset condition, wherein the prompt information is used to prompt the user to enter the first set instruction through the user interface.

3. The sample analysis system of any one of claims 1 to 2, **characterized in that** the processor is further configured to find a target image from the at least two first images, wherein a preset parameter value of the target image is the largest among the at least two first images, and is greater than a preset threshold; and
the display screen is further configured to display the target image on the user interface.

4. The sample analysis system of claim 3, **characterized in that** the processor is further configured to control the display screen to display the three-dimensional representative in rotation on the user interface if the target image cannot be found from the at least two first images.

5. The sample analysis system of claim 3 or 4, **characterized in that** the preset parameter value represents a ratio of a first area to a second area, the first area representing an area of a first image that is occupied by the blood sample, and the second area representing an area of said first image that is occupied by the sample container.

6. The sample analysis system of any one of claims 1 to 5, **characterized in that** the display screen outputting the animation on the user interface comprises:
displaying a preset image frame of the animation on the user interface after playing of the animation is paused or completed.

7. The sample analysis system of claim 6, **characterized in that** a first parameter value of the preset image frame is greater than a first preset threshold, wherein
the first parameter value represents a ratio of a first area to a second area, the first area representing an area of the preset image frame that is occupied by the blood sample, and the second area representing an area of the preset image frame that is occupied by the sample container.

8. The sample analysis system of claim 6 or 7, **characterized in that** at least one image parameter of the preset image frame meets a preset parameter requirement, wherein the at least one image parameter comprises at least one of the following:
brightness;
sharpness; and
contrast.

9. The sample analysis system of any one of claims 6 to 8, **characterized in that** the processor is further configured to:
control the display screen to stop playing the animation on the user interface, in response to a third set instruction entered by the user through the user interface; and/or
control the display screen to display image frames of the animation frame by frame or in a frame skipping manner on the user interface, in response to a fourth set instruction entered by the user through the user interface.

10. The sample analysis system of any one of claims 6 to 9, **characterized in that** the processor generating, based on the at least two first images, an animation for displaying from different angles the sample container containing the blood sample comprises:
performing frame interpolation on the at least two first images, to obtain image frames for forming the animation.

11. The sample analysis system of claim 10, **characterized in that** the processor performing frame interpolation on the at least two first images comprises:
performing first image processing on each first image, to obtain at least two second images; performing frame interpolation on the at least two second images, to obtain at least three second images; and performing second image processing on each of the at least three second images, to obtain the image frames for forming the animation, wherein
the first image processing is used at least to remove a surface curvature of the sample container in each first image, and the second image processing is used at least to recover said surface curvature of the sample container in each second image.

12. The sample analysis system of any one of claims 1 to 13, **characterized in that** the processor is further configured to generate, as the three-dimensional representative, a rotatable three-dimensional image or three-dimensional model of the sample container containing the blood sample based on the at least two first images.

13. The sample analysis system of any one of claims 1 to 12, **characterized in that** the image obtaining module comprises a camera, a rotation mechanism, and a gripping mechanism, wherein
the gripping mechanism is configured to grip the sample container, to keep the sample container in a vertical state;
the rotation mechanism is configured to drive the gripping mechanism to rotate, to drive the sample container gripped by the gripping mechanism to rotate about a vertical direction; and
the camera is configured to capture the at least two first images of the sample container during rotation.

14. The sample analysis system of claim 13, **characterized in that** the gripping mechanism comprises:
a base configured to grip a bottom of the sample container; and/or
a manipulator configured to grip a top of the sample container.

15. A sample information display method, comprising:
obtaining at least two first images, which are different from each other, of a sample container loaded with a blood sample, wherein each first image at least partially displays at least part of the blood sample in the sample container, and the at least two first images are obtained by imaging the sample container before an analysis device performs a sample analysis on the blood sample;
generating, based on the at least two first images, a rotatable three-dimensional representative of the sample container containing the blood sample and/or an animation for displaying from different angles the sample container containing the blood sample; and
**characterized by** comprising:
controlling a display screen to display an analysis result obtained by the analysis device by analyzing the blood sample in the sample container, and to output the rotatable three-dimensional representative and/or the animation, on a user interface;
in response to a first set instruction inputted by a user via the user interface, controlling the display screen to display the three-dimensional representative in rotation on the user interface, so as to display the sample container containing the blood sample from a plurality of lateral angles; and/or in response to a second set instruction entered by the user through the user interface, controlling the display screen to play the animation on the user interface, so as to display the sample container containing the blood sample from a plurality of lateral angles.

## Patentansprüche

1. Ein Probenanalysesystem, umfassend ein Eingabemodul, eine Transportbahn, eine Analysevorrichtung, ein Bildaufnahmemodul, einen Prozessor und einen Bildschirm,
wobei das Eingabemodul so ausgelegt ist, dass es einen mit einer Blutprobe befüllten Probenbehälter trägt und den Probenbehälter auf die Transportbahn überführt;
die Transportbahn dazu ausgelegt ist, den Probenbehälter zur Analysevorrichtung zu befördern;
die Analysevorrichtung dazu ausgelegt ist, die Blutprobe im Probenbehälter zu analysieren, um ein Analyseergebnis zu erhalten;
das Bildaufnahmemodul ist so ausgelegt, dass es mindestens zwei voneinander unterschiedliche erste Bilder des Probenbehälters aufnimmt, bevor die Analysevorrichtung eine Probenanalyse an der Blutprobe durchführt, wobei jedes erste Bild zumindest teilweise mindestens einen Teil der Blutprobe im Probenbehälter zeigt;
der Prozessor ist so ausgelegt, dass er auf der Grundlage der mindestens zwei ersten Bilder ein drehbares dreidimensionales Modell des die Blutprobe enthaltenden Probenbehälters und/oder eine Animation zur Darstellung des die Blutprobe enthaltenden Probenbehälters aus verschiedenen seitlichen Blickwinkeln erzeugt; und
**dadurch gekennzeichnet, dass**:
der Anzeigebildschirm ist dazu konfiguriert, das Analyseergebnis anzuzeigen und den drehbaren dreidimensionalen Repräsentanten und/oder die Animation auf einer Benutzeroberfläche auszugeben;
wobei der Prozessor weiterhin dazu konfiguriert ist: als Reaktion auf eine erste Set-Anweisung, die von einem Benutzer über die Benutzeroberfläche eingegeben wurde, den Bildschirm so zu steuern, dass die dreidimensionale Darstellung auf der Benutzeroberfläche rotierend angezeigt wird, um den Probenbehälter, der die Blutprobe enthält, aus mehreren seitlichen Blickwinkeln darzustellen, und/oder der Prozessor ist ferner so konfiguriert, dass er: als Reaktion auf einen zweiten Einstellungsbefehl, der vom Benutzer über die Benutzeroberfläche eingegeben wurde, den Bildschirm so zu steuern, dass die Animation auf der Benutzeroberfläche abgespielt wird, um den Probenbehälter, der die Blutprobe enthält, aus mehreren seitlichen Blickwinkeln darzustellen.

2. Das Probenanalysesystem nach Anspruch 1, **dadurch gekennzeichnet, dass** der Prozessor ferner so konfiguriert ist, dass er:
den Bildschirm so zu steuern, dass auf der Benutzeroberfläche eine Aufforderungsmeldung ausgegeben wird, wenn das Analyseergebnis eine voreingestellte Bedingung nicht erfüllt, wobei die Aufforderungsmeldung dazu dient, den Benutzer aufzufordern, den ersten Einstellungsbefehl über die Benutzeroberfläche einzugeben.

3. Das Probenanalysesystem nach einem der Ansprüche 1 bis 2, **dadurch gekennzeichnet, dass** der Prozessor ferner so konfiguriert ist, dass er aus den mindestens zwei ersten Bildern ein Zielbild ermittelt, wobei ein voreingestellter Parameterwert des Zielbildes der größte unter den mindestens zwei ersten Bildern ist und größer als ein voreingestellter Schwellenwert ist; und dass der Bildschirm ferner so konfiguriert ist,
dass er das Zielbild auf der Benutzeroberfläche anzeigt.

4. Das Probenanalysesystem nach Anspruch 3, **dadurch gekennzeichnet, dass** der Prozessor ferner so konfiguriert ist, dass er den Bildschirm so steuert, dass die dreidimensionale Darstellung auf der Benutzeroberfläche rotierend angezeigt wird, falls das Zielbild nicht aus den mindestens zwei ersten Bildern ermittelt werden kann.

5. Das Probenanalysesystem nach Anspruch 3 oder 4, **dadurch gekennzeichnet, dass** der voreingestellte Parameterwert ein Verhältnis eines ersten Bereichs zu einem zweiten Bereich darstellt, wobei der erste Bereich einen Bereich eines ersten Bildes darstellt, der von der Blutprobe eingenommen wird, und der zweite Bereich einen Bereich des ersten Bildes darstellt, der vom Probenbehälter eingenommen wird.

6. Das Probenanalysesystem nach einem der Ansprüche 1 bis 5, **dadurch gekennzeichnet, dass** der Bildschirm, der die Animation auf der Benutzeroberfläche ausgibt, Folgendes umfasst:
die Anzeige eines voreingestellten Bildes der Animation auf der Benutzeroberfläche, nachdem die Wiedergabe der Animation angehalten oder beendet wurde.

7. Das Probenanalysesystem nach Anspruch 6, **dadurch gekennzeichnet, dass** ein erster Parameterwert des voreingestellten Bildrahmens größer ist als ein erster voreingestellter Schwellenwert, wobei
der erste Parameterwert ein Verhältnis eines ersten Bereichs zu einem zweiten Bereich darstellt, wobei der erste Bereich einen Bereich des voreingestellten Bildrahmens darstellt, der von der Blutprobe eingenommen wird, und der zweite Bereich einen Bereich des voreingestellten Bildrahmens darstellt, der vom Probenbehälter eingenommen wird.

8. Das Probenanalysesystem nach Anspruch 6 oder 7, **dadurch gekennzeichnet, dass** mindestens ein Bildparameter des voreingestellten Bildrahmens eine voreingestellte Parameteranforderung erfüllt, wobei der mindestens eine Bildparameter mindestens einen der folgenden Parameter umfasst:
Helligkeit;
Schärfe; und
Kontrast.

9. Das Probenanalysesystem nach einem der Ansprüche 6 bis 8, **dadurch gekennzeichnet, dass** der Prozessor ferner so konfiguriert ist, dass er:
den Bildschirm so zu steuern, dass die Wiedergabe der Animation auf der Benutzeroberfläche als Reaktion auf einen dritten Einstellungsbefehl, der vom Benutzer über die Benutzeroberfläche eingegeben wurde, beendet wird; und/oder
den Bildschirm so zu steuern, dass Bildrahmen der Animation auf der Benutzeroberfläche Bild für Bild oder unter Überspringen von Bildrahmen angezeigt werden, als Reaktion auf einen vierten Einstellungsbefehl, der vom Benutzer über die Benutzeroberfläche eingegeben wurde.

10. Das Probenanalysesystem nach einem der Ansprüche 6 bis 9, **dadurch gekennzeichnet, dass** der Prozessor, der auf der Grundlage der mindestens zwei ersten Bilder eine Animation zur Darstellung des die Blutprobe enthaltenden Probenbehälters aus verschiedenen Blickwinkeln erzeugt, Folgendes umfasst:
Durchführung einer Bildinterpolation an den mindestens zwei ersten Bildern, um Bildrahmen zur Erstellung der Animation zu erhalten.

11. Das Probenanalysesystem nach Anspruch 10, **dadurch gekennzeichnet, dass** der Prozessor, der eine Bildinterpolation an den mindestens zwei ersten Bildern durchführt, Folgendes umfasst:
Durchführung einer ersten Bildverarbeitung an jedem ersten Bild, um mindestens zwei zweite Bilder zu erhalten; Durchführung einer Bildinterpolation an den mindestens zwei zweiten Bildern, um mindestens drei zweite Bilder zu erhalten; und Durchführung einer zweiten Bildverarbeitung an jedem der mindestens drei zweiten Bilder, um die Bildrahmen zur Erzeugung der Animation zu erhalten, wobei
Die erste Bildverarbeitung dient zumindest dazu, in jedem ersten Bild eine Oberflächenkrümmung des Probenbehälters zu beseitigen, und die zweite Bildverarbeitung dient zumindest dazu, in jedem zweiten Bild die genannte Oberflächenkrümmung des Probenbehälters wiederherzustellen.

12. Das Probenanalysesystem nach einem der Ansprüche 1 bis 13, **dadurch gekennzeichnet, dass** der Prozessor ferner so konfiguriert ist, dass er auf der Grundlage der mindestens zwei ersten Bilder als dreidimensionale Darstellung ein drehbares dreidimensionales Bild oder ein dreidimensionales Modell des die Blutprobe enthaltenden Probenbehälters erzeugt

13. Das Probenanalysesystem nach einem der Ansprüche 1 bis 12, **dadurch gekennzeichnet, dass** das Bildaufnahmemodul eine Kamera, einen Drehmechanismus und einen Greifmechanismus umfasst, wobei
der Greifmechanismus so ausgelegt ist, dass er den Probenbehälter ergreift, um den Probenbehälter in einer vertikalen Lage zu halten;
der Drehmechanismus so ausgelegt ist, dass er den Greifmechanismus zur Drehung antreibt, um den vom Greifmechanismus ergriffenen Probenbehälter um eine vertikale Achse zu drehen; und
Die Kamera ist so konfiguriert, dass sie die mindestens zwei ersten Bilder des Probenbehälters während der Drehung aufnimmt.

14. Das Probenanalysesystem nach Anspruch 13, **dadurch gekennzeichnet, dass** der Greifmechanismus umfasst:
eine Basis, die so ausgelegt ist, dass sie einen Boden des Probenbehälters ergreift; und/oder
einen Manipulator, der so ausgelegt ist, dass er eine Oberseite des Probenbehälters ergreift.

15. Verfahren zur Anzeige von Probeninformationen, umfassend:
das Erfassen von mindestens zwei ersten Bildern, die sich voneinander unterscheiden, eines mit einer Blutprobe beladenen Probenbehälters, wobei jedes erste Bild zumindest teilweise mindestens einen Teil der Blutprobe im Probenbehälter zeigt und die mindestens zwei ersten Bilder durch Abbilden des Probenbehälters erfasst werden, bevor eine Analysevorrichtung eine Probenanalyse an der Blutprobe durchführt;
erzeugen, basierend auf den mindestens zwei ersten Bildern, eines drehbaren dreidimensionalen Repräsentanten des Probenbehälters, der die Blutprobe enthält, und/oder
einer Animation zum Anzeigen des Probenbehälters, der die Blutprobe enthält, aus verschiedenen Winkeln; und
**gekennzeichnet dadurch, dass** es Folgendes umfasst:
Steuern eines Anzeigebildschirms zum Anzeigen eines Analyseergebnisses, das das Analysegerät durch Analysieren der Blutprobe im Probenbehälter erhalten hat, und zum Ausgeben des drehbaren dreidimensionalen Repräsentanten und/oder der Animation auf einer Benutzeroberfläche;
als Reaktion auf einen eingegebenen ersten Set-Befehldurch einen Benutzer über die Benutzeroberfläche Steuern des Anzeigebildschirms, um den dreidimensionalen Repräsentanten in Rotation auf der Benutzeroberfläche anzuzeigen, um so den Probenbehälter, der die Blutprobe enthält, aus mehreren seitlichen Winkeln anzuzeigen; und/oder als Reaktion auf einen zweiten Einstellungsbefehl, der vom Benutzer über die Benutzeroberfläche eingegeben wurde, die Steuerung des Bildschirms, um die Animation auf der Benutzeroberfläche abzuspielen, um den die Blutprobe enthaltenden Probenbehälter aus einer Vielzahl von seit len Blickwinkeln darzustellen.

## Revendications

1. Système d'analyse d'échantillon, comprenant un module d'entrée, une piste de transfert, un dispositif d'analyse, un module d'obtention d'image, un processeur, et un écran d'affichage, où
le module d'entrée est configuré pour supporter un contenant d'échantillon chargé avec un échantillon de sang, et transférer le contenant d'échantillon vers la piste de transfert ;
la piste de transfert est configurée pour transférer le contenant d'échantillon vers le dispositif d'analyse ;
le dispositif d'analyse est configuré pour analyser l'échantillon de sang dans le contenant d'échantillon, afin d'obtenir un résultat d'analyse ;
le module d'obtention d'image est configuré pour acquérir au moins deux premières images du contenant d'échantillon, lesquelles sont différentes l'une de l'autre, avant que le dispositif d'analyse ne réalise une analyse d'échantillon sur l'échantillon de sang, où chaque première image affiche au moins partiellement au moins une partie de l'échantillon de sang dans le contenant d'échantillon ;
le processeur est configuré pour générer, sur la base des au moins deux premières images, une représentation tridimensionnelle pivotable du contenant d'échantillon contenant l'échantillon de sang et/ou une animation afin d'afficher selon différents angles latéraux le contenant d'échantillon contenant l'échantillon de sang ; et
**caractérisé en ce que** :
l'écran d'affichage est configuré pour afficher le résultat d'analyse et fournir la représentation tridimensionnelle pivotable et/ou l'animation sur une interface utilisateur ;
où le processeur est en outre configuré pour : en réponse à une première instruction définie entrée par un utilisateur par le biais de l'interface utilisateur, commander à l'écran d'affichage d'afficher la représentation tridimensionnelle en rotation sur l'interface utilisateur, de sorte à afficher le contenant d'échantillon contenant l'échantillon de sang selon une pluralité d'angles latéraux, et/ou le processeur est en outre configuré pour : en réponse à une deuxième instruction définie entrée par l'utilisateur par le biais de l'interface utilisateur, commander à l'écran d'affichage de lire l'animation sur l'interface utilisateur, de sorte à afficher le contenant d'échantillon contenant l'échantillon de sang selon une pluralité d'angles latéraux.

2. Système d'analyse d'échantillon selon la revendication 1, **caractérisé en ce que** le processeur est en outre configuré pour :
commander à l'écran d'affichage de fournir une information d'invite sur l'interface utilisateur lorsque le résultat d'analyse ne satisfait pas à une condition prédéfinie, où l'information d'invite est utilisée pour inviter l'utilisateur à entrer la première instruction définie par le biais de l'interface utilisateur.

3. Système d'analyse d'échantillon selon l'une quelconque des revendications 1 et 2, **caractérisé en ce que** le processeur est en outre configuré pour trouver une image cible parmi les au moins deux premières images, où une valeur de paramètre prédéfinie de l'image cible est la plus grande parmi les au moins deux premières images, et est supérieure à un seuil prédéfini ; et
l'écran d'affichage est en outre configuré pour afficher l'image cible sur l'interface utilisateur.

4. Système d'analyse d'échantillon selon la revendication 3, **caractérisé en ce que** le processeur est en outre configuré pour commander à l'écran d'affichage d'afficher la représentation tridimensionnelle en rotation sur l'interface utilisateur si l'image cible ne peut pas être trouvée parmi les au moins deux premières images.

5. Système d'analyse d'échantillon selon la revendication 3 ou 4, **caractérisé en ce que** la valeur de paramètre prédéfinie représente un rapport d'une première aire sur une seconde aire, la première aire représentant une aire d'une première image qui est occupée par l'échantillon de sang, et la seconde aire représentant une aire de ladite première image qui est occupée par le contenant d'échantillon.

6. Système d'analyse d'échantillon selon l'une quelconque des revendications 1 à 5, **caractérisé en ce que** l'écran d'affichage fournissant l'animation sur l'interface utilisateur comprend :
le fait d'afficher un cadre d'image prédéfini de l'animation sur l'interface utilisateur après que la lecture de l'animation est interrompue ou achevée.

7. Système d'analyse d'échantillon selon la revendication 6, **caractérisé en ce qu'**une première valeur de paramètre du cadre d'image prédéfini est supérieure à un premier seuil prédéfini, où
la première valeur de paramètre représente un rapport d'une première aire sur une seconde aire, la première aire représentant une aire du cadre d'image prédéfini qui est occupée par l'échantillon de sang, et la seconde aire représentant une aire du cadre d'image prédéfini qui est occupée par le contenant d'échantillon.

8. Système d'analyse d'échantillon selon la revendication 6 ou 7, **caractérisé en ce qu'**au moins un paramètre d'image du cadre d'image prédéfini respecte une exigence de paramètre prédéfinie, où le au moins un paramètre d'image comprend au moins l'un des éléments suivants :
luminosité ;
netteté ; et
contraste.

9. Système d'analyse d'échantillon selon l'une quelconque des revendications 6 à 8, **caractérisé en ce que** le processeur est en outre configuré pour :
commander à l'écran d'affichage d'arrêter la lecture de l'animation sur l'interface utilisateur, en réponse à une troisième instruction définie entrée par l'utilisateur par le biais de l'interface utilisateur ; et/ou
commander à l'écran d'affichage d'afficher des cadres d'image de l'animation cadre par cadre ou d'une manière par saut de cadre sur l'interface utilisateur, en réponse à une quatrième instruction définie entrée par l'utilisateur par le biais de l'interface utilisateur.

10. Système d'analyse d'échantillon selon l'une quelconque des revendications 6 à 9, **caractérisé en ce que** le processeur générant, sur la base des au moins deux premières images, une animation afin d'afficher selon différents angles le contenant d'échantillon contenant l'échantillon de sang comprend :
le fait de réaliser une interpolation de cadre sur les au moins deux premières images, afin d'obtenir des cadres d'image destinés à former l'animation.

11. Système d'analyse d'échantillon selon la revendication 10, **caractérisé en ce que** le processeur réalisant une interpolation de cadre sur les au moins deux premières images comprend :
le fait de réaliser un premier traitement d'image sur chaque première image, afin d'obtenir au moins deux secondes images ; le fait de réaliser une interpolation de cadre sur les au moins deux secondes images, afin d'obtenir au moins trois secondes images ; et le fait de réaliser un second traitement d'image sur chacune des au moins trois secondes images, afin d'obtenir des cadres d'image destinés à former l'animation, où
le premier traitement d'image est utilisé au moins pour supprimer une incurvation de surface du contenant d'échantillon sur chaque première image, et le second traitement d'image est utilisé au moins pour récupérer ladite incurvation de surface du contenant d'échantillon sur chaque seconde image.

12. Système d'analyse d'échantillon selon l'une quelconque des revendications 1 à 13, **caractérisé en ce que** le processeur est en outre configuré pour générer, en tant que représentation tridimensionnelle, une image tridimensionnelle pivotable ou un modèle tridimensionnel pivotable du contenant d'échantillon contenant l'échantillon de sang sur la base des au moins deux premières images.

13. Système d'analyse d'échantillon selon l'une quelconque des revendications 1 à 12, **caractérisé en ce que** le module d'obtention d'image comprend un appareil de prise de vues, un mécanisme de rotation, et un mécanisme de préhension, où
le mécanisme de préhension est configuré pour tenir le contenant d'échantillon, afin de garder le contenant d'échantillon dans un état vertical ;
le mécanisme de rotation est configuré pour amener le mécanisme de préhension à tourner, pour amener le contenant d'échantillon tenu par le mécanisme de préhension à tourner autour d'une direction verticale ; et
l'appareil de prise de vues est configuré pour acquérir les au moins deux premières images du contenant d'échantillon durant la rotation.

14. Système d'analyse d'échantillon selon la revendication 13, **caractérisé en ce que** le mécanisme de préhension comprend :
une base configurée pour tenir un fond du contenant d'échantillon ; et/ou
un dispositif de manipulation configuré pour tenir un sommet du contenant d'échantillon.

15. Procédé d'affichage d'informations d'échantillon, comprenant les étapes consistant à :
obtenir au moins deux premières images, lesquelles sont différentes l'une de l'autre, d'un contenant d'échantillon chargé avec un échantillon de sang, où chaque première image affiche au moins partiellement au moins une partie de l'échantillon de sang dans le contenant d'échantillon, et les au moins deux premières images sont obtenues en formant en image le contenant d'échantillon avant qu'un dispositif d'analyse ne réalise une analyse d'échantillon sur l'échantillon de sang ;
générer, sur la base des au moins deux premières images, une représentation tridimensionnelle pivotable du contenant d'échantillon contenant l'échantillon de sang et/ou une animation afin d'afficher selon différents angles le contenant d'échantillon contenant l'échantillon de sang ; et
**caractérisé par** le fait de comprendre les étapes consistant à :
commander à un écran d'affichage d'afficher un résultat d'analyse obtenu par le dispositif d'analyse en analysant l'échantillon de sang dans le contenant d'échantillon, et de fournir la représentation tridimensionnelle pivotable et/ou l'animation sur une interface utilisateur ;
en réponse à une première instruction définie entrée par un utilisateur par le biais de l'interface utilisateur, commander à l'écran d'affichage d'afficher la représentation tridimensionnelle en rotation sur l'interface utilisateur, de sorte à afficher le contenant d'échantillon contenant l'échantillon de sang selon une pluralité d'angles latéraux ; et/ou en réponse à une deuxième instruction définie entrée par l'utilisateur par le biais de l'interface utilisateur, commander à l'écran d'affichage de lire l'animation sur l'interface utilisateur, de sorte à afficher le contenant d'échantillon contenant l'échantillon de sang selon une pluralité d'angles latéraux.
